(19) **Europäisches Patentamt**
**European Patent Office**
**Office européen des brevets**

(11) **EP 1 998 730 B1**

(12) **FASCICULE DE BREVET EUROPEEN**

(45) Date de publication et mention
de la délivrance du brevet:
**03.07.2019 Bulletin 2019/27**

(21) Numéro de dépôt: **07731803.8**

(22) Date de dépôt: **23.03.2007**

(51) Int Cl.:
*A61F 13/02* (2006.01)     *A61F 13/08* (2006.01)

(86) Numéro de dépôt international:
**PCT/FR2007/050990**

(87) Numéro de publication internationale:
**WO 2007/113430 (11.10.2007 Gazette 2007/41)**

(54) **NOUVEAU SYSTEME DE CONTENTION POUR LE TRAITEMENT ET LA PREVENTION DES PATHOLOGIES DES MALADIES VEINEUSES**

NEUES STÜTZSYSTEM ZUR BEHANDLUNG UND PRÄVENTION VON VENENERKRANKUNGEN

NOVEL SUPPORT SYSTEM FOR TREATMENT AND PREVENTION OF DISEASES OF THE VEINS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **24.03.2006 FR 0651025**

(43) Date de publication de la demande:
**10.12.2008 Bulletin 2008/50**

(60) Demande divisionnaire:
**13156061.7 / 2 818 145**
**13156175.5 / 2 617 400**
**13156223.3 / 2 818 146**

(73) Titulaire: **Urgo Recherche Innovation et Développement**
**21300 Chenove (FR)**

(72) Inventeurs:
• **LECOMTE, Serge**
**21000 Dijon (FR)**
• **DUPASQUIER, Guy**
**42340 Rivas (FR)**
• **VERMEULEN, Catherine**
**42270 Saint Priest En Jarez (FR)**

(74) Mandataire: **Hubert, Philippe et al**
**Cabinet Beau de Loménie**
**158, rue de l'Université**
**75340 Paris Cedex 07 (FR)**

(56) Documents cités:
**EP-A- 1 709 947**      **EP-A2- 0 878 179**
**WO-A-00/42957**      **US-A1- 2004 199 096**
**US-A1- 2005 192 524**

**Description**

La présente invention concerne un nouveau système de contention pour le traitement et la prévention des pathologies d'origine veineuse.

Ce système constitué de deux couches est facile et rapide à poser.

L'utilisation de divers systèmes de contention est connue depuis longtemps pour traiter les pathologies d'origine veineuse, comme par exemple l'insuffisance veineuse, le traitement des varices et les ulcères de jambes, ou encore pour prévenir la thrombose veineuse.

En effet, l'application d'une pression adéquate agit favorablement :

- d'une part, au niveau des vaisseaux en réduisant le calibre des veines ce qui conduit à l'accélération du flux sanguin et au rétablissement de la fonction valvulaire ; et
- d'autre part, au niveau des tissus en favorisant une meilleure oxygénation et une résorption de l'oedème.

Dans le traitement des plaies chroniques et tout particulièrement dans celui des ulcères de jambes, l'utilisation d'un système de contention qui permet de rétablir ou de favoriser une circulation veineuse normale, est le traitement de référence et la seule thérapie qui ait prouvé son efficacité pour soigner et éviter la récidive de ce type de plaies.

Un système de contention efficace doit permettre de répondre à quatre objectifs principaux.

Premièrement, le système doit pouvoir être porté de façon continue jour et nuit durant plusieurs jours (par exemple une semaine).

A cet effet, ce système doit donc permettre d'appliquer simultanément :

- d'une part, une pression relativement faible appelée « pression de repos », quand le muscle est relâché pour être confortable et en particulier supportable durant la nuit ; et
- d'autre part, une pression relativement élevée appelée « pression de travail », quand le muscle est tendu ou lors des mouvements, et en particulier pendant la marche.

Deuxièmement, le différentiel de pression entre pression de repos et pression de travail doit être suffisant pour favoriser le reflux veineux.

Troisièmement, les valeurs de pression au repos, de pression de travail et de différentiel de pression doivent être stables dans le temps.

Quatrièmement, le système doit être facile et rapide à poser, et ceci en toute sécurité, afin d'éviter les risques de garrot si la pression appliquée est trop élevée, ou d'inefficacité si la pression ou le différentiel de pression est trop faible.

Pour parvenir à ces objectifs, on a développé différents systèmes utilisant des bandes élastiques tricotées ou tissées appelées bandes de contention.

Lors de son application autour d'un membre comme la jambe, chaque bande est étirée et suivant le degré d'allongement, elle applique sur la jambe une pression plus ou moins élevée.

Cette pression, qui est la pression de traitement, dépend principalement de deux facteurs, l'extensibilité de la bande et la circonférence du membre sur lequel cette dernière est appliquée.

Les bandes élastiques de contention sont donc enroulées à une extension donnée autour de la jambe. Lors de l'enroulement de la bande, on procède à un recouvrement plus ou moins total de celle-ci sur elle-même. Très souvent, ce recouvrement est de 50 %.

Les fabricants fournissent généralement des tableaux et des gammes de produits qui, pour un diamètre de membre donné, permettent de déterminer la pression à appliquer et de choisir le système adéquat.

Les bandes de contention sont classées de façon très imprécise par les professionnels de santé en deux grandes catégories.

### 1) Les bandes dites à allongement court

Ces bandes sont destinées à être posées à faible allongement (par exemple souvent défini comme inférieur à 70 % à 90%) et exercent une pression au repos basse et une pression de travail élevée. Elles ont donc une grande amplitude de pression en particulier lors des mouvements, par exemple pendant la marche.

Les bandes à allongement court possèdent toutefois de nombreux inconvénients.

Tout d'abord, elles sont difficiles à poser car de faibles variations d'allongement à la pose génèrent une forte augmentation ou diminution de la pression atteinte. Il existe donc un risque de garrot si la pression appliquée est trop élevée ou un risque d'inefficacité si elle est insuffisante.

Par ailleurs, leur faible extensibilité rend leur manipulation difficile (en particulier le passage du talon et la remontée en spirales à recouvrement à 50 %). De plus, posée à un allongement proche du seuil de verrouillage de la bande, c'est à dire proche de la limite à partir de laquelle tout allongement supplémentaire provoque son endommagement irréversible, la bande est souvent sujette à une déformation importante au cours du temps ce qui entraîne un risque de perte de ses propriétés élastiques. Il en résulte la nécessité de procéder à des changements fréquents de ces bandes et une augmentation corrélative du coût du traitement. De même, en cas d'oedème en cours de traitement, le diamètre du membre diminue lors de la phase de réduction de l'oedème et pour éviter tout risque de garrot, ces bandes doivent être remplacées fréquemment.

2) Les bandes à allongement long

Ces bandes sont plus faciles à poser car elles présentent une meilleure extensibilité. De ce fait, des variations d'allongement à la pose ne génèrent pas de variations importantes de la pression appliquée. Le risque de garrot est faible.

En revanche, ces bandes conduisent à de faibles variations de pression entre repos et travail et à une faible variation de pression au cours des mouvements, par exemple lors de la marche.

Elles présentent aussi un certain inconfort en position de repos si l'on souhaite imposer une pression élevée, d'où la nécessité de les retirer la nuit en raison de la gêne occasionnée.

Ces deux catégories de bandes sont utilisées seules ou en association avec une bande d'ouate destinée à répartir les pressions à la surface du membre, et/ou à protéger les saillies osseuses grâce à son épaisseur, et à absorber les éventuels exsudats si la bande est posée sur une plaie ouverte, par exemple dans le cas des ulcères de jambes.

Des bandes à allongement long sont par exemple commercialisées par les sociétés 3M, THUASNE et SMITH ET NEPHEW respectivement sous les dénominations Coheban®, Biflex® et Proguide®.

Des bandes à allongement court en association avec une bande d'ouate sont par exemple commercialisées par la société ACTIVA sous la dénomination Actico®.

De tels systèmes sont par exemple commercialisés par les sociétés SMITH AND NEPHEW et URGO Limited respectivement sous les dénominations Profore® et K4.

Ces systèmes à quatre couches comprennent :

- une première couche interne de ouate qui remplit les mêmes fonctions que précédemment décrites ;
- une deuxième couche qui est une bande de crêpe très faiblement élastique qui sert d'interface et permet de niveler la surface de la couche de ouate ;
- une troisième couche qui est une bande très élastique, c'est à dire à allongement long, destinée à appliquer une partie de la pression totale au membre ; et
- une quatrième couche qui est une bande très élastique à allongement long, auto adhérente, destinée à appliquer le complément de pression, et à maintenir l'ensemble du système en place.

En théorie, ce système applique une pression de repos basse donc sans inconfort, une pression de travail élevée et par conséquent un différentiel de pression efficace.

Toutefois, l'utilisation de couches multiples rend ce système peu économique, très encombrant et long à poser.

L'utilisation des deux dernières couches, qui sont des bandes élastiques qui possèdent des extensibilités élevées, favorise la sécurité vis à vis des risques de garrot. En revanche, la pose de ces couches successives, sans véritable contrôle de l'extensibilité, rend incertaine l'efficacité réelle du système lors de sa mise en oeuvre. L'augmentation du nombre de couches augmente aussi le risque de glissement du système. Enfin, la variabilité des propriétés élastiques inhérente à leur fabrication rend encore plus incertaine l'efficacité réelle du système.

Le document EP 0 878 179 propose un système de contention à trois couches comprenant :

- une première couche constituée d'une bande de mousse de polyuréthane liée par aiguilletage à une couche absorbante ;
- une deuxième couche constituée d'une bande élastique tricotée ;
- une troisième couche constituée d'une bande élastique auto-adhérente.

Un tel système présente les mêmes inconvénients que le système à quatre couches discuté précédemment en raison de l'utilisation de deux bandes élastiques.

Le document US 2005/0192524 décrit un système de contention comportant une pluralité de bandes pouvant être placées sur la jambe et fixées à l'aide d'attaches de type « Velcro® ».

Ces bandes peuvent être munies d'un dispositif visuel permettant d'évaluer la pression appliquée.

Tous les systèmes de contention connus à ce jour visent principalement, par l'action d'une ou plusieurs bandes élastiques en association avec une ouate absorbante, à appliquer sur la jambe une pression cible de l'ordre de 30 à 50 mmHg pour une cheville moyenne de l'ordre de 23 à 25 cm de circonférence, et à maintenir ces pressions au cours du temps.

Cependant, il apparaît que les notions de différentiel de pression et de mouvement n'ont pas été étudiées par les fabricants de ces produits connus.

Tous ces systèmes ont été conçus en partant du seul principe selon lequel la pression appliquée répond à la formule définie par la loi de Laplace :

$$P = k\frac{2\Pi F}{C} \times n$$

dans laquelle :

P est la pression exprimée en mm de mercure,
F est la force de tension, exprimée en Newtons/cm, de la bande à un allongement donné,
C est la circonférence, exprimée en cm, du membre sur lequel la (ou les) bande (s) est (sont) enroulée (s),
n est le nombre de spires formées par la bande autour du membre ; et k est une constante.

Toutefois, la loi de Laplace correspond à un modèle mathématique statique. Son utilisation pour élaborer ou choisir un système de contention efficace ne prend donc pas en compte les aspects dynamiques liés à l'évolution et l'adaptation du système en fonction des variations rapides de la circonférence de la jambe lors des mouvements. Des mesures de pression in vivo réalisées à l'aide de capteurs de pression disposés sous un système de contention quatre couches ont montré qu'un tel système peut effectivement fournir un différentiel de pression efficace lors des mouvements. On ignore cependant véritablement pourquoi et quelles sont les caractéristiques qui permettent d'obtenir ce résultat.

Par ailleurs, les systèmes de contention connus utilisant une ouate en association avec une ou plusieurs bandes élastiques ne prennent pas en compte les forces de frottement et de compression qui s'appliquent entre ces différentes couches, ni les variations de diamètre qui résultent inévitablement de la pose successive des différentes couches.

Diverses études ont montré que la pression totale exercée par le système n'est pas proportionnelle à la somme des pressions exercées par chaque bande. Les inventeurs ont constaté également que la couche de ouate a une influence importante sur les valeurs de la pression appliquée par une bande.

Si l'on ajoute à ces contraintes dynamiques les problèmes de pose, qui influencent les propriétés d'efficacité et d'innocuité des systèmes de contention cités précédemment, il existe donc une très grande incertitude sur les performances réelles de ces systèmes ce qui explique sans doute les résultats contradictoires ou décevants obtenus lors de différentes études cliniques.

Quelques solutions ont été proposées pour tenter d'améliorer les systèmes de contention. On peut ainsi citer les documents brevets EP 490 793, EP 671 898, EP 820 261, EP 876 809 et US 3 613 679 qui décrivent des moyens d'étalonnage permettant de sécuriser et faciliter la pose des bandes élastiques.

On a décrit également dans le brevet EP 460 040 une bande élastique qui fournit une force de compression adéquate à de faibles allongements, ladite force de compression restant quasi constante même en cas de forte augmentation de l'allongement.

Enfin, le brevet EP 651 628 décrit un système de contention monocouche résultant de l'association d'une bande élastique auto adhérente et d'une couche absorbante afin d'obtenir un bandage qui permette d'appliquer une force compressive permanente quand il est allongé. La couche absorbante est destinée à protéger la plaie en absorbant les forces de compression et à absorber les exsudats.

Toutefois, le produit décrit dans ce brevet antérieur présente de nombreux inconvénients.

Si la ouate recouvre l'ensemble de la surface de la bande élastique auto adhérente, ce système ne peut tenir seul. De même, si une partie seulement de la bande auto adhérente est recouverte par la couche absorbante lors de l'enroulement du bandage autour du membre, on augmente les risques de glissement d'une couche sur l'autre et par conséquent d'évolution de la pression appliquée au cours du temps lors des mouvements. Un recouvrement non homogène est la source de variations importantes des pressions appliquées.

Même si l'utilisation d'un tel système de contention constitué d'une seule couche peut permettre de diminuer l'influence de la couche de ouate, elle ne permet pas de résoudre tous les problèmes liés à l'obtention d'un système de contention efficace et facile à poser.

Dans ces conditions, il apparaît qu'aucun des documents précités ni aucune des solutions décrites à ce jour n'envisage d'étudier ou d'évaluer l'influence des contraintes dynamiques liées aux mouvements sur les propriétés à obtenir pour mettre au point un système de contention efficace.

C'est l'approche de ce problème qui a permis la réalisation de la présente invention, en déterminant les caractéristiques essentielles que doivent posséder un système de contention efficace ainsi que les bandes qui le composent.

L'objet de la présente invention est donc de fournir un système de contention qui permette de s'affranchir des interférences de la ouate sur la pression appliquée, qui réponde aux contraintes dynamiques et à leurs conséquences sur l'efficacité globale lors de son utilisation, qui soit facile à poser, confortable et qui maintienne un différentiel de pression efficace au cours du temps.

Afin d'obtenir un tel système, les inventeurs ont étudié la variation des différences de pressions entre pression de repos et de travail en particulier lors des mouvements, et leurs évolutions au cours du temps.

Les inventeurs ont ainsi analysé les propriétés des systèmes de contention connus, en mesurant en particulier les pressions à la pose, le différentiel de pression lors des mouvements, les pressions de contention maximum (travail) et minimum (repos) et leurs évolutions dans le temps.

Ils ont ainsi pu mettre en évidence les propriétés des systèmes connus à allongement court, à allongement long et des systèmes multicouches ainsi que l'importance de la couche de ouate et retrouver les résultats rapportés sur ces produits lors de tests cliniques in vivo par des mesures de pression sous le système de contention.

Par ailleurs, et d'une façon surprenante, l'étude dynamique des propriétés viscoélastiques des bandes a permis de déterminer une nouvelle caractéristique physique, le coefficient d'élasticité dynamique, qui est corrélée avec les différences de pression.

En fait, les bandes de contention sont des matériaux viscoélastiques dont les caractéristiques techniques résultent du choix de leurs constituants, les fils (denier, nature des fibres, guipage), leur construction textile (tissage, tricotage) et des traitements supplémentaires qui leurs sont appliqués (vaporisation, incorporation de latex auto adhérent, adhésivation, etc.).

Les caractéristiques techniques de ces bandes sont traduites par leurs courbes de traction-rupture, bien connues par les spécialistes des matériaux textiles.

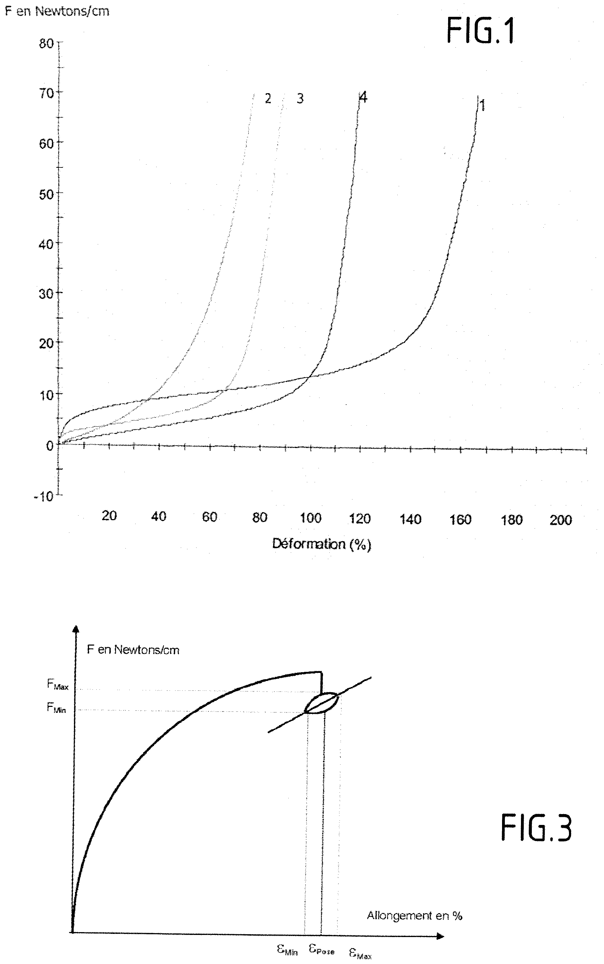

La relation entre la force (et donc la pression appliquée) et l'allongement de la bande est illustrée par la représentation graphique d'un essai de traction-allongement d'un échantillon de bande à l'aide d'une machine d'essais à la traction. Cette représentation fait apparaître les différentes zones caractéristiques de déformation d'une bande élastique et peut être considérée comme la « carte d'identité » de chaque bande. Les types de courbes obtenues à l'aide de cet essai pour divers produits commercialisés sont ainsi représentés sur la figure 1.

Les inventeurs ont étudié, pour la première fois, l'évolution des caractéristiques de divers systèmes de contention et de leurs constituants pour un allongement donné sur la courbe de traction-rupture, c'est à dire l'évolution dynamique de ces systèmes et de leurs constituants autour d'un point de fonctionnement. C'est en étudiant cette évolution que les inventeurs ont mis en évidence, d'une façon tout à fait inattendue et surprenante, une caractéristique dynamique (qu'ils ont dénommée coefficient d'élasticité dynamique) et qu'ils ont découvert qu'il existe une corrélation, pour une bande élastique donnée, entre cette caractéristique et le différentiel de pression procuré par cette bande, évalué in vitro. De plus, il a été découvert que, dans le cas de la superposition de deux bandes de contention, la somme des différences de pression du système est quasi égale à la somme des différences de pression générées par chaque bande et que de plus, il existe le même phénomène d'addition pour les coefficients d'élasticité dynamique. Cette corrélation est totalement inattendue. En effet, elle relie deux grandeurs expérimentales obtenues selon des méthodes d'essais qui sont complètement différentes dans leur principe. En outre, elle est vérifiée avec des produits de propriétés (allongement, élasticité) et de structure textile (tissus, tricot, latexification...) très différentes. Enfin, malgré les variations intrinsèques liées à la reproductibilité de fabrication de ces bandes élastiques et aux incertitudes sur les mesures de différentiel de pression et du coefficient d'élasticité dynamique, les corrélations obtenues sont excellentes.

Grâce à ces éléments, et après avoir éliminé les interférences introduites par la présence d'une couche de ouate indépendante en choisissant un système de contention dans lequel cette couche de ouate est liée à une bande élastique, les inventeurs ont pu déterminer les caractéristiques que doivent remplir un système optimisé se présentant sous la forme nouvelle d'un système bicouche, ainsi que chacune de ses couches, pour être efficace et facile à mettre en oeuvre.

En d'autres termes, les études entreprises par les inventeurs ont rendu possible l'élaboration d'un nouveau système de contention bicouche qui permet de résoudre pour la première fois dans l'état de la technique, l'ensemble des problèmes mentionnés précédemment.

La présente invention propose donc un nouveau système de contention bicouche exerçant, entre une position de repos dans laquelle le muscle est relâché et une position de travail dans laquelle le muscle est tendu, un différentiel de pression compris entre 10 et 40 mm de mercure, caractérisé en ce qu'il comprend :

- une première couche de contention constituée d'une bande élastique liée sur l'ensemble de sa surface à une couche absorbante , ladite couche absorbante étant constituée d'un matériau textile tissé ou non tissé à base de fibres, et ladite bande élastique étant constituée d'un matériau textile tissé, non tissé ou d'un tricot;
- une deuxième couche de contention constituée d'une bande élastique auto adhérente;
- lesdites première et seconde couches étant en outre pourvues d'un système d'étalonnage indiquant l'allongement à la pose respectif de chacune de ces deux couches et choisi de telle sorte que :

le coefficient d'élasticité dynamique desdites première et deuxième couche soit compris entre 1 et 45 Newtons/cm et que

le coefficient d'élasticité dynamique du système de contention soit compris entre 5 et 55 Newtons/cm;

le coefficient d'élasticité dynamique étant mesuré selon la description;

- lesdites première et deuxième couche présentant en outre une rémanence à leur allongement à la pose inférieure à 10 %.

Selon un mode de réalisation préféré de la présente invention, le coefficient d'élasticité dynamique du système est compris entre 10 et 35 et de préférence 15 et 30 Newtons/cm.

Selon un autre mode de réalisation préféré de la présente invention, la première et /ou la deuxième couche présente une rémanence à son allongement à la pose comprise entre 4 et 8 %.

## Description détaillée de l'invention

L'invention sera mieux comprise et d'autres caractéristiques apparaîtront plus clairement à la lecture de la description explicative qui va suivre faite en référence aux dessins annexés dans lesquels :

- la figure 1 représente les courbes traction-rupture de quatre produits connus, commercialisés par les sociétés THUASNE, ACTIVA et SMITH et NEPHEW respectivement sous les dénominations BIFLEX® 16 (3), ACTICO® (2), PROGUIDE® (1) et PROFORE 4®(4) ;
- la figure 2 est une vue en coupe transversale illustrant un appareil de mesure de pression et de différentiel de pression in vitro ;
- la figure 3 illustre le principe de la mesure du coefficient d'élasticité dynamique d'une bande ;
- la figure 4 illustre le principe de la mesure du coefficient d'élasticité dynamique d'un système de deux bandes de contention selon l'invention ;
- la figure 5 illustre le principe des tests traction-allongement et de mesure de la force et de la rémanence d'une bande à un allongement donné ;
- la figure 6 illustre la corrélation entre les différentiels de pression $\Delta$ exprimés en mm de mercure et les coefficients d'élasticité dynamique appelés ci-après CED, exprimés en Newtons par cm, pour l'ensemble des bandes individuelles et des systèmes de contention réalisés selon l'invention ;
- la figure 7 illustre la corrélation entre les mêmes paramètres, mais uniquement pour les systèmes de contention réalisés ;
- la figure 8 illustre la corrélation entre la somme des différentiels de pression $\Delta$ de chaque bande individuelle constituant un système de contention et les différentiels de pression $\Delta$ des systèmes de contention correspondants selon l'invention ;
- la figure 9 illustre la corrélation entre la somme des CED de chaque bande constituant un système de contention et les CED des systèmes de contention correspondants selon l'invention ; et
- la figure 10 illustre l'évolution au cours du temps, exprimé en jours, du différentiel de pression $\Delta$ (exprimé en mm de mercure) pour les produits ACTICO® (ouate + bande auto adhérente posée à son allongement maximum) (courbe 2) ; PROFORE® dont les quatre couches sont respectivement posées aux allongements 0 % (ouate), 35 %, 50 % et 50 % (courbe 4) ainsi que pour deux systèmes de contention constitués des éléments suivants Ex2-40 % + Ex4-50 % (courbe 3) et Ex1-30 % + Ex6-90 % (courbe 1).

La présente invention permet, en s'affranchissant du rôle de la ouate et grâce au choix du coefficient d'élasticité dynamique de chaque bande et du système de contention dans son ensemble, de mettre à disposition du personnel soignant une solution efficace, simple de mise en oeuvre et confortable pour le patient, permettant de traiter toutes les pathologies liées à l'insuffisance veineuse. En outre, en permettant de jouer principalement sur les coefficients d'élasticité dynamique, et subsidiairement sur la rémanence des bandes utilisées et sur les possibilités d'étalonnage, la présente invention offre, pour la première fois dans l'état de la technique, la possibilité d'adapter le choix des deux couches constituant le système de contention, à la durée du traitement, à la gravité de la pathologie, en particulier en présence d'oedèmes ou d'ulcères de jambe par exemple. Dans tous les cas, cette adaptation se traduit par des gains appréciables en termes de temps de pose et donc de coûts et par une meilleure sécurité à la pose car la détermination du CED permet de lever l'incertitude liée à la reproductibilité des propriétés élastiques et dynamiques des bandes utilisées. La grande flexibilité dans l'association des deux bandes qui découle de la présente invention, permet ainsi de s'adapter à de multiples situations.

La possibilité de répartir le différentiel de pression sur les deux bandes permet en effet de garantir la possibilité de mettre au point en toute sécurité des systèmes de contention offrant une large gamme de différentiels de pression. On considère généralement qu'un différentiel de pression compris entre 15 mm et 25 mm de mercure est nécessaire pour rétablir un flux veineux correct. Toutefois selon la pathologie, qu'il s'agisse d'un traitement sur des jambes sans ulcère grave ou d'un traitement difficile sur des jambes très abîmées avec oedème, cette plage de valeurs peut s'étendre de 10 à 35 mm de mercure voire même de 10 à 40 mm de mercure. L'invention permet, par exemple, d'appliquer sur une jambe en bonne santé, une pression de travail et un différentiel de pression plus élevé dans la mesure où les risques de garrot ou les risques d'endommager les tissus de la jambe sont plus faibles qu'avec les systèmes connus, d'autant plus encore que la pose est facilitée.

L'un des objectifs de la présente invention est de fournir un système de contention permettant d'assurer un différentiel de pression compris de préférence entre 15 et 25 mm de mercure voire entre 10 et 35 mm de mercure, voire même encore entre 10 et 40 mm de mercure si nécessaire.

Pour atteindre cet objectif, on pourra donc jouer sur le coefficient d'élasticité dynamique de chaque bande afin d'obtenir ainsi un système de contention ayant un coefficient d'élasticité dynamique qui varie entre 5 et 55 Newtons/cm.

De préférence, on utilisera un système de contention qui présentera un coefficient d'élasticité dynamique compris entre 10 et 35 Newtons/cm et plus particulièrement entre 15 et 30 Newtons/cm. Dans cette dernière plage de valeurs, il a été constaté que l'on obtient un différentiel de pression qui se stabilise rapidement et évolue peu ou n'évolue pas au cours du temps.

Pour atteindre ces coefficients d'élasticité dynamique, les première et deuxième couches du système de contention selon l'invention, seront formées de bandes dont le coefficient d'élasticité dynamique est compris entre 1 et 45 Newtons/cm, de préférence entre 3 et 30 Newtons/cm et de préférence encore entre 5 et 25 Newtons/cm.

Afin de faciliter la pose des bandes qui forment les deux couches du système de contention et d'obtenir un différentiel de pression stable rapidement ainsi qu'au cours du temps, on utilisera des bandes qui présentent en outre une rémanence mesurée à leur allongement à la pose inférieure à environ 10 % et de préférence comprise entre 4 et 8 %.

Un tel mode de réalisation est particulièrement adapté dans le cas de pathologies habituellement difficiles à traiter.

On a en effet constaté que de telles bandes sont plus faciles à poser, en particulier si leur extensibilité est faible ou pour un allongement proche du seuil de verrouillage. De plus, on a constaté que de telles bandes subissent après la pose, une chute de pression et de différentiel de pression très faible au cours du temps. On diminue ainsi les risques d'inconfort à la pose. Avec de telles bandes on obtient, dès la pose, les valeurs de pression de travail, de repos et de différentiel de pression requises. On a déterminé que pour des bandes possédant une rémanence inférieure à 10 %, ces valeurs évoluent peu, voire même n'évoluent pas au cours de temps, si la rémanence est comprise entre 4 et 8 %.

Par ailleurs, afin de favoriser une pose précise par le personnel soignant, chaque bande formant le système de contention de la présente invention sera munie d'un moyen d'étalonnage.

Ce moyen d'étalonnage peut être visuel, comme par exemple un ensemble de pictogrammes, régulièrement espacés, imprimés sur la bande ou réalisés au moyen d'un système d'étalonnage comme par exemple un pochoir par le personnel soignant, à qui l'on donne des informations sur les allongements à la pose recommandés. Ce type de pochoir ou les explications nécessaires pour le fabriquer peuvent être incorporés dans un kit qui comprend les deux bandes de contention qui forment le système de contention ou un assortiment de bandes de contention qui permet de réaliser différents systèmes de contention adéquats.

Le principe de l'étalonnage à l'aide d'un pochoir est le suivant.

Le pochoir est en général réalisé au moyen d'une feuille carton dans laquelle a été découpée une ouverture qui peut avoir, par exemple, une forme ellipsoïdale ou rectangulaire, permettant de réaliser, comme on le comprend, un pictogramme de même forme sur la bande.

La transformation de ces pictogrammes en cercle ou en carré sous l'effet de l'allongement de la bande permet ainsi d'étalonner cette dernière. Ce principe est décrit par exemple dans le brevet américain US 3 613 679.

On utilise de préférence la forme rectangulaire qui est plus facile à découper dans une feuille en carton.

La longueur et la largeur du rectangle sont déterminées mathématiquement en fonction de l'allongement auquel on souhaite poser la bande. Ainsi si $E$ est l'allongement de la bande à la pose et $L$ la longueur du rectangle qui sera parallèle au sens de la trame de la bande (c'est-à-dire perpendiculaire au sens de la longueur de la bande), la largeur $L$a du rectangle sera $\dfrac{L}{1+E}$ . Par exemple, pour étalonner une bande que l'on souhaite poser à 55 % d'allongement on utilisera un rectangle ayant 4 cm de longueur une largeur de 4/(1 + 0,55) soit 2,58 cm.

Pour étalonner la bande on découpe dans un pochoir en carton des rectangles de 4 cm de long et de 2,58 cm de large. Ce pochoir est disposé sur la bande à plat non étirée, et l'on trace sur la bande, par exemple à l'aide d'un feutre, la périphérie de ce rectangle.

Dans le cadre de la présente invention, on préférera l'utilisation de bandes préalablement étalonnées lors de la fabrication à l'aide de pictogrammes sous forme d'ellipses qui se transforment en cercle à l'allongement souhaité. Selon l'invention, on utilisera un système de contention dont les deux couches constitutives sont étalonnées.

Un des principaux avantages de la présente invention est de permettre, dans la mesure où elles entrent dans le domaine de coefficient d'élasticité dynamique, un grand choix dans les bandes utilisables pour obtenir un système de contention efficace.

Une bande élastique absorbante utilisable comme première couche dans le système de contention de la présente invention peut être fabriquée selon des techniques bien connues de l'homme du métier. La réalisation de tels produits est par exemple décrite dans le brevet EP 651 628 dans lequel on associe une bande élastique auto adhérente et une couche absorbante.

Dans le cadre de la présente invention, il n'est pas nécessaire que la bande élastique soit obligatoirement auto adhérente. Elle peut ne pas l'être, voire ne l'être que sur une seule face, par exemple celle opposée à la couche absorbante.

La couche absorbante peut être fixée par tous moyens connus sur la bande élastique pour réaliser la première couche du système de contention selon l'invention. Elle doit toutefois recouvrir toute la surface de la bande élastique.

Des technologies textiles variées, comme par exemple le faufilage ou l'aiguilletage, la soudure par ultrasons, le complexage ou la fixation à l'aide d'un adhésif, peuvent être utilisées pour réaliser cette première couche.

Dans le cadre de la présente invention, on préférera utiliser une couche absorbante constituée d'une ouate et fixer cette dernière à la bande élastique soit par aiguilletage soit à l'aide d'un adhésif. Cet adhésif peut, par exemple, être un latex de même nature que celui qui sert à cohésiver les bandes élastiques pour les rendre auto adhérentes ou un adhésif du type acrylique.

Les couches absorbantes utilisables dans le cadre de l'invention, sont choisies parmi les matériaux textiles tissés ou non tissés à base de fibres synthétiques ou naturelles, comme par exemple les ouates.

Dans le cadre de la présente invention, on préférera l'utilisation comme couche absorbante de matériaux textiles tissés ou non tissés à base de fibres qui présentent une meilleure conformabilité que les mousses.

Ces matériaux textiles grâce à leur structure discontinue due à l'assemblage des fibres présentent une surface déformable qui leur permet de mieux s'adapter aux courbures et aux parties saillantes des membres à recouvrir que les mousses qui présentent une surface plane continue plus rigide.

De même, grâce à leur structure discontinue, les matériaux textiles en général présentent une perméabilité à la vapeur d'eau plus importante que les mousses pour des épaisseurs équivalentes.

A titre d'exemple, les ouates peuvent être constituées de fibres de viscoses, de polyester, polyuréthanne, coton, rayonne, etc.

De telles ouates sont par exemple les produits commercialisés par les sociétés URGO Limited, ACTIVA ou SMITH et NEPHEW respectivement sous les dénominations K-SOFT®, FLEXI-BAN® et SOFT-BAN®.

Dans le cadre de la présente invention, on préférera tout particulièrement la ouate K-SOFT® qui est constituée d'un mélange de 60 % de fibres viscose et 40 % de fibres polyester.

Si on désire augmenter ou favoriser la capacité d'absorption de la couche absorbante, on utilisera avantageusement des ouates à base de fibres super absorbantes. De telles ouates sont par exemple décrites dans le brevet EP 0 873 097.

La couche absorbante pourra contenir éventuellement des agents actifs qui contribuent à l'amélioration de la cicatrisation de l'ulcère de jambe ou qui permettent de diminuer la douleur ou l'oedème, ou encore des agents antibactériens.

Selon une variante de réalisation, on pourra introduire dans la ouate des fibres antibactériennes, par exemple des fibres argent, ou imprégner celle-ci avec un antibactérien par exemple du triclosan.

Selon une autre variante de réalisation, on pourra encore déposer un agent antibactérien, comme par exemple un composé à base d'argent, à la surface de la couche absorbante ou sur l'ensemble de la première couche du système (couche absorbante-bande élastique).

Tout type de bande élastique peut être utilisé pour la fabrication de la première couche du système de contention selon l'invention, qu'elle soit tissée, non tissée ou tricotée. On préférera cependant un tissu.

Dans le cadre de la présente invention, l'ensemble bande élastique-couche absorbante formant la première couche doit toutefois présenter la caractéristique essentielle qui permet d'obtenir un système de contention efficace, à savoir un coefficient d'élasticité dynamique compris entre 1 et 45 Newtons/cm.

En effet, en dessous de 1 Newton/cm cette première couche n'apporte aucune valeur de pression ni de différentiel. Elle confère alors au système un comportement équivalent à celui d'une ouate.

Au-delà de 45 Newtons/cm, on a observé que la force qu'elle subit est très importante et la pression qu'elle exerce diminue très rapidement de même que le différentiel initial de pression, quel que soit la nature de la deuxième couche. On a constaté en effet que la présence de la deuxième couche ne modifie pas le comportement de la première couche si celle-ci a un coefficient d'élasticité dynamique trop élevé et inversement. Même si une telle bande atteint un équilibre au bout de quelques heures en termes de pressions appliquées et de différentiel de pression, le risque d'imposer une pression trop élevée au départ et de chute de pression du système au cours du temps est trop important.

La deuxième couche du système de contention selon l'invention est constituée d'une bande élastique auto-adhérente qui présente un coefficient d'élasticité dynamique compris entre 1 et 45 Newtons/cm.

Une bande élastique auto adhérente utilisable comme deuxième couche peut être de nature variée.

D'une façon générale, on utilisera des bandes élastiques qui sont revêtues de latex de caoutchouc naturel ou d'adhésifs ayant un collant faible.

La réalisation de telles bandes est bien connue de l'homme du métier.

Le latex est de préférence déposé sur les deux côtés de la bande élastique à raison environ de 0,01 à 75 % en poids afin de fournir une bande élastique qui soit adhérente sur elle-même sans adhérer à la peau ou aux vêtements. Dans le cadre de la présente invention, on préférera des enductions de latex de l'ordre de 25 à 33 g/m².

Divers types de constructions textiles qui peuvent être utilisées pour réaliser des bandes élastiques auto adhérentes sont par exemple décrits dans le brevet EP 651 628

Des procédés permettant de déposer du latex ou des adhésifs ayant un faible collant sur des bandes élastiques sont décrits par exemple dans les brevets US 4 699 133 et FR 2 611 756.

Des bandes élastiques auto adhérentes commercialisées par exemple par les sociétés 3M, ACTIVA, URGO Limited et SMITH ET NEPHEW respectivement sous les dénominations COHEBAN®, ACTICO®, KO-FLEX® et PROFORE 4® sont aussi utilisables dans le cadre de la présente invention.

On décrira maintenant les méthodes permettant d'évaluer les propriétés d'un système de contention selon l'invention et de déterminer les caractéristiques essentielles que doivent remplir les bandes qui le constituent.

L'étude des propriétés dynamiques in vitro des systèmes de contention a pu être réalisée grâce à la mise au point d'un appareillage spécifique.

Son principe est le suivant :

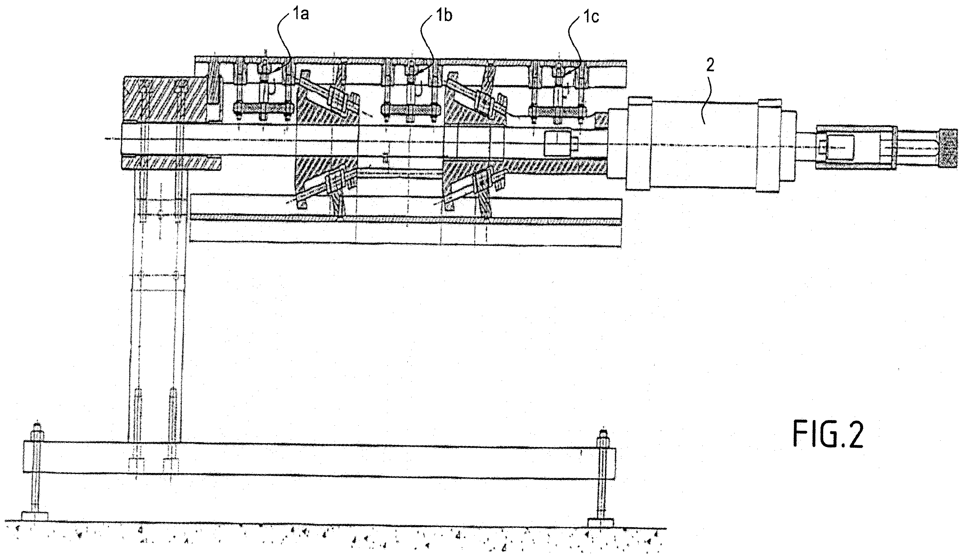

Sur un dispositif cylindrique tel qu'illustré à la figure 2, on applique une succession de cycles, qui consistent à faire varier la circonférence du dispositif cylindrique, à une amplitude et une vitesse paramétrables.

La variation de circonférence est commandée par un système pneumatique. La fréquence des cycles est liée au paramétrage du système pneumatique. Le niveau de variation de circonférence choisi est de l'ordre de 3 % ce qui correspond à la valeur de déformation moyenne de la circonférence du muscle de la jambe lorsqu'il se contracte. Une série de trois capteurs à jauges de contraintes (1a, 1b, 1c) est fixée au dispositif et procède à la mesure en temps réel des pressions. L'ensemble de ces données est transmis via un logiciel d'acquisition à un ordinateur où un traitement ultérieur peut être effectué.

Le dispositif est généralement constitué de quatre plaques d'acier identiques, lisses, cintrées et inscrites dans un cylindre. Ces quatre plaques sont solidaires de deux systèmes d'appui coniques dont l'un est fixe et l'autre peut être mis en mouvement de translation sous l'action d'un vérin (2) actionné par un système pneumatique.

Sous l'action du vérin, les deux systèmes d'appui coniques renvoient un mouvement de translation qui permet aux quatre plaques de s'écarter de l'axe d'une distance proportionnelle, cette distance étant réglée par une commande fixe en extrémité du vérin.

Sur l'une des quatre plaques, sont ménagées trois fenêtres dans lesquelles sont logés trois capteurs à jauge de contrainte (traction/compression) XTFC commercialisés par la société DOERLER sur lesquels a été fixée une surface affleurant de même courbure que la plaque. Les caractéristiques techniques de ces capteurs sont :

- Plage 0-10 Newtons, linéarité ≤ 0,5 % de l'étendue maximum
- Hystérésis ≤ 0,5 % de l'étendue maximum

Les capteurs électroniques sont conditionnés et raccordés à une centrale d'acquisition trois voies développée par la société DOERLER avec un convertisseur analogique numérique laquelle est en communication via un logiciel avec un ordinateur pour le suivi dans le temps et le traitement des données.

Ce cylindre possède un périmètre minimum de 35,9 cm et un périmètre maximum de 37 cm ce qui correspond à la déformation moyenne de 3 % choisie pour correspondre à la variation moyenne de la circonférence de la jambe entre repos et travail et lors de la marche. Afin de se rapprocher encore plus de la réalité, on pose à t = 0 la bande à 75 % de cette déformation, soit sur un cylindre qui a une circonférence de 36,2 cm. En effet, lors de la pose d'une bande, la jambe n'est pas totalement au repos. Le système est étalonné par étalonnage des capteurs en statique grâce à une bande rigide à laquelle on a suspendu deux poids. Pour réaliser les mesures, les systèmes et les bandes de contention sont enroulés autour de ce cylindre avec un seul recouvrement de 100 % pour les tests réalisés ci-après.

On découpe une longueur de bande nécessaire pour obtenir ce recouvrement. Dans le cas où l'on teste une bande seule qui n'est pas auto adhérente, cette dernière est fixée à l'allongement souhaité à l'aide d'un ruban adhésif. La pose à l'allongement souhaité peut être aussi facilitée par la présence d'un moyen d'étalonnage sous forme de pictogrammes sur la bande.

Afin d'optimiser encore les conditions de pose, on utilise de plus la méthode suivante.

L'appareil présente quatre repères gradués espacés d'une distance $d_1$ égale au périmètre du cylindre à la pose (36,2 cm) divisé par 4 soit 9,05 cm. On fabrique un gabarit sur une feuille rigide présentant des graduations espacées

d'une dimension $d_2$ telle que si $E$ est l'allongement à la pose de la bande, la graduation $d_2$ est telle qu'elle vérifie la relation $d2 = d_1/(1 + E)$. Les repères $d_2$ du gabarit sont reportés sur la bande au repos. La bande est ensuite appliquée sur l'appareil de telle sorte que les repères reportés sur la bande coïncident avec ceux ($d_1$) présents sur l'appareil. Dans

$$E = \frac{d1 - d2}{d2}.$$

cette position on a l'allongement souhaité puisque

La mesure du coefficient d'élasticité dynamique est effectuée selon la méthode suivante, dont le principe est illustré par la figure 3.

Le protocole d'essai consiste en une série de cycles de traction charge / décharges au voisinage d'une extension donnée (déformation $\varepsilon_{pose}$) et d'amplitude faible (quelques pourcents) autour de ce point (variation : $\varepsilon_{max}$ - $\varepsilon_{min}$).

Dans les tests qui ont été réalisés, la variation a été fixée à 3 %.

La mise en oeuvre du test est réalisée sur une machine de traction qui est un dynamomètre électronique, par exemple un dynamomètre de marque MTS, équipé d'un capteur de 100 Newtons. La bande est conditionnée à 21 $\pm$ 2°C et une hygrométrie de 60 $\pm$ 15 % d'humidité relative pendant 24 heures. Le test est réalisé dans les mêmes conditions. On découpe un échantillon de bande de 10 cm de largeur et de 20 cm de longueur pour effectuer le test. Afin d'éviter les incertitudes de mesures liées à la relaxation des matériaux textiles après découpe, on laisse l'échantillon de bande se reposer 10 min avant d'effectuer la mesure. L'échantillon est ensuite monté entre les mâchoires du dynamomètre électronique puis étiré jusqu'à l'extension à la pose ($\varepsilon_{pose}$) à la vitesse de 500 mm/min. Une période de relaxation de 30 secondes précède une série de cinq cycles charge / décharge d'amplitude $\varepsilon_{max}$ - $\varepsilon_{min}$ autour de ce point à la vitesse 100 mm/min. Par définition, le coefficient d'élasticité dynamique dénommé ci-après CED est le résultat de l'équation suivante :

$$CED = \left( \frac{F_{max} - F_{min}}{\varepsilon_{max} - \varepsilon_{min}} \right)_5$$

On mesure ce coefficient au terme du cinquième cycle.

Il s'exprime en N/cm.

$F_{max}$ et $F_{min}$ sont les forces qui correspondent aux variations d'allongement $\varepsilon_{max}$ et $\varepsilon_{min}$.

Un des avantages de cette technique est qu'elle permet aussi de déterminer expérimentalement le CED sur une paire de bandes.

On a constaté que le CED équivalent à une paire de bandes est égal à la somme des CED de chaque bande. Cette relation est aussi vérifiée expérimentalement si les deux bandes sont sollicitées à des niveaux d'extension différents. Dans ce cas, les deux bandes sont maintenues toutes les deux dans les mâchoires du dynamomètre, mais la bande qui est tendue à la plus faible extension est insérée dans les mâchoires du dynamomètre avec une longueur initiale plus importante.

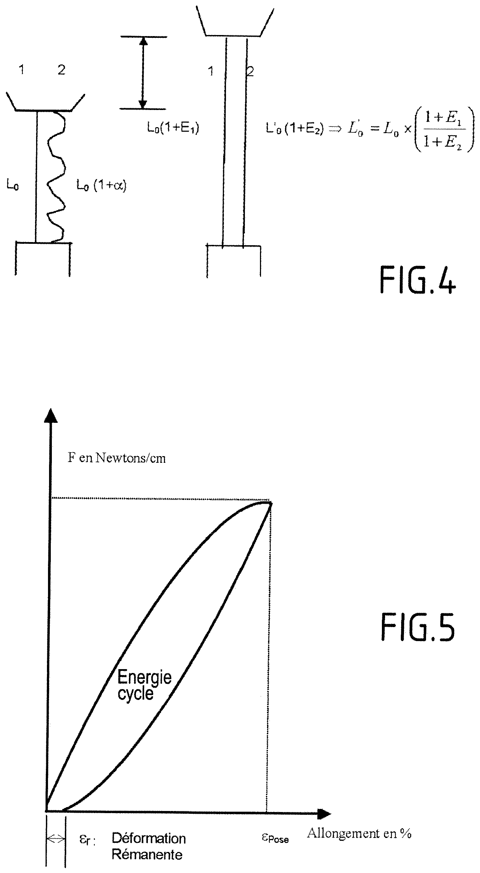

La mesure du CED de deux bandes dont le principe est illustré sur la figure 4, est réalisée de la façon suivante.

Dans cette figure, $L_0$ représente la longueur initiale de l'éprouvette d'une première bande destinée à être étirée avec un taux d'allongement $E_1$.

De même, $L'_0$ représente la longueur de l'éprouvette de la deuxième bande destinée à être étirée à un taux d'allongement $E_2$ inférieur à $E_1$.

Connaissant les taux d'allongement $E_1$ et $E_2$, il est aisé de déterminer la longueur $L'_0$ de l'éprouvette de la seconde bande, celle-ci répondant à la formule suivante :

$$L'_0 = L_0 \times \left[ \frac{1 + E_1}{1 + E_2} \right]$$

Par exemple, pour réaliser la sollicitation en parallèle de deux bandes destinées à être étirées respectivement à des taux d'allongement de 50 % et de 40 %, on utilisera une éprouvette de longueur $L'_0$ = à 214 mm pour la bande destinée à être étirée à un allongement de 40 % si l'on choisit une éprouvette de longueur $L_0$ = à 200 mm pour la bande destinée à être étirée à un taux d'allongement de 50 %.

Enfin, les propriétés élastiques des bandes formant les couches d'un système de contention selon la présente invention ont été étudiées selon deux autres tests classiques, les courbes traction-allongement et la rémanence. Ces deux tests sont illustrés sur la figure 5. Ils servent aussi avec le grammage à caractériser les produits fabriqués dans les exemples de réalisation.

Le test de traction-allongement utilise la même machine de traction (dynamomètre de la marque MTS) que précédemment équipée d'un capteur 100 Newtons.

On soumet un échantillon de bande élastique de 20 cm de longueur et 10 cm de largeur à l'allongement auquel on souhaite réaliser la mesure de force à une vitesse de 100 mm par minute.

Le test est réalisé à une température de 21 $\pm$ 2° C et une hygrométrie de 60 $\pm$ 15 % d'humidité relative.

On mesure sur la courbe obtenue la force à l'allongement visé qui est exprimée en Newtons par centimètre.

On décrira maintenant le principe du test de la mesure de la rémanence.

Ce test utilise la même machine de traction dans les mêmes conditions que précédemment.

On soumet la bande à un cycle charge-décharge jusqu'à l'allongement souhaité de la bande ($\varepsilon_{pose}$). On détermine ainsi, à l'issue du cycle, la rémanence ou déformation rémanente $\varepsilon_r$ comme illustré sur la figure 5.

Cette rémanence qui est un allongement est exprimée en pourcentage.

On donnera maintenant divers exemples de systèmes de contention conformes à la présente invention.

Différentes premières couches utilisables dans les systèmes de contention de la présente invention ont été fabriquées de la façon suivante :

1) Fabrication de la ouate

La ouate est un non tissé qui est fabriqué par le procédé classique de cardage, nappage et préaiguilletage à partir de fibres viscose et de fibres polyester dans la proportion 60 % - 40 % en poids.

Ces fibres ont les caractéristiques suivantes :

- Fibres de viscose blanches sans azurant optique de 1,7 dtex et 50 mm de longueur ;
- Fibres de polyester blanches sans azurant optique de 3,3 dtex et 60 mm de longueur.

Cette ouate présente un grammage de 75 g par m$^2$.

La même ouate a été utilisée en association avec différentes bandes élastiques pour la fabrication des premières couches du système de contention selon l'invention.

2) Fabrication de la bande élastique

a) Pour réaliser la première couche des systèmes de contention selon l'invention, on a utilisé différents tricots qui ont été fabriqués sur un métier Raschel jauge 12 et dont les caractéristiques techniques sont les suivantes :

**Tricot 1** :

Chaîne :

- Fil polyamide commercialisé par la société RADICI sous la référence 78/24/1SZ ;
- alimentation : 48 fils pour 10 cm.
- Fil élasthanne nu commercialisé par la société FILLATTICE spa sous la référence LINEL 78 dtex ;
- alimentation : 48 fils pour 10 cm.
  Ce fil est soumis à une tension de 8 cNewtons pour deux fils.

Trame :

- Fil polyamide commercialisé sous la référence 78/24/1SZ.
- Le débit de trame est de 4,6.

Duitage : 9,3 duites par cm.

**Tricot 2** :

Chaîne :

- Fil de polyamide référence 78/24/1SZ identique au précédent ;
- alimentation : 48 fils pour 10 cm.
- Fil d'élasthanne sous référence LINEL 78 dtex,
- alimentation : 48 fils pour 10 cm.
  Ce fil est soumis à une tension de 8 cNewtons pour deux fils.

Trame :

- Fil polyamide référence 78/24/1SZ.
- Le débit de trame est de 4,9 .

Duitage : 9,3 duites par cm

**Tricot 3** :

Chaîne :

- Fil de viscose Nm 40 ;
- alimentation : 49 fils pour 10 cm.
- Fil d'élasthanne sous référence LINEL 78 dtex ;
- alimentation : 49 fils pour 10 cm.
  Ce fil est soumis à une tension de 12 cNewtons pour deux fils

Trame :

- Fil de viscose Nm 40.
- Le débit de trame est de 3,7 .

Duitage : 7,6 duites par cm

3) Fabrication de la première couche par aiguilletage

Le procédé général de fabrication par aiguilletage est classique.
La ouate fabriquée précédemment est déroulée et insérée sous l'aiguilleteuse avec un des tricots fabriqués précédemment qui est soumis à une extension de l'ordre de 70 à 100 %.
L'aiguilletage est réalisé avec une planche à aiguilles comprenant 15000 aiguilles pour une laize de 2,5 à 2,8 mètres. En sortie d'aiguilleteuse, on obtient l'ensemble ouate-tricot que l'on découpe en laizes de 10 cm que l'on enroule sans tension. Ces laizes sont ensuite coupées pour donner les bandes élastiques de longueur souhaitée qui servent de première couche dans le système de contention de la présente invention.
Les caractéristiques des bandes obtenues avec les tricots 1 à 3 sont les suivantes :

**Exemple 1 :**

- Grammage : 230 g/m$^2$
- Force à 40 % d'allongement : 1 Newton/cm
- Rémanence : 4,4 %

**Exemple 2** :

- Grammage                  : 200 g/m$^2$
- Force à 50 % d'allongement  : 0,9 Newton/cm
- Rémanence             : 7,1 %

**Exemple 3 :**

- Grammage                  : 210 g/m$^2$
- Force à 40 % d'allongement  : 0,6 Newton/cm
- Rémanence             : 4,5 %

4) <u>Fabrication de la deuxième couche</u>

Différentes bandes élastiques auto adhérentes utilisables en tant que deuxième couche du système de contention de la présente invention ont été fabriquées sur un métier à tisser de type jet d'air de marque TSUDAKOMA®.

Lors du tissage, on applique une tension de l'ordre de 30 cNewtons par fil d'élasthanne.

Après le tissage, le produit est vaporisé jusqu'à sa rétractation totale.

On réalise ensuite une enduction de latex (produit commercialisé par la société SIKA France sous la référence SIKACOLL-2057/00) de l'ordre de 30 g par m$^2$ de produit tissé pour conférer à la bande élastique son caractère auto adhérent.

On réalise ainsi des bandes de 10 cm de largeur. Les caractéristiques techniques des différentes bandes ainsi réalisées sont les suivants :

**Exemple 4 :**

Chaîne :

- Fil polyamide de 1/78 dex

- Fil élasthanne guipé air jet polyamide commercialisé par la société Moulinages SCHWARZENBACH sous la référence PE 635SEJ

Trame :
- Fil acrylique couleur chair NM 34/1

| | |
|---|---|
| Duitage : | 10 duites par centimètre |
| Grammage : | 225 g/m$^2$ |
| Force à 50 % d'allongement : | 0,5 Newton/cm |
| Rémanence : | 4% |

**Exemple 5 :**

Chaîne :

- Fil polyamide de 2/78 dex

- Fil élasthanne guipé polyamide commercialisé par la société Moulinages SCHWARZENBACH sous la référence PE 9403NE

Trame:
- Fil acrylique couleur chair NM 34/1

| | |
|---|---|
| Duitage : | 17 duites par cm |
| Grammage : | 230 g |
| Force à 50 % d'allongement : | 1,5 Newtons/cm |
| Rémanence : | 3% |

**Exemple 6 :**

Chaîne :

- Fil polyamide de 2/78 dtex

- Fil élasthanne guipé air jet polyamide commercialisé par la société Moulinages SCHWARZENBACH sous la référence PE 459SEJ

Trame : Fil acrylique couleur chair NM 34/1

| | |
|---|---|
| Duitage : | 10 duites par cm |
| Grammage : | 175 g/m$^2$ |
| Force à 80 % d'allongement : | 4,3 Newtons/cm |
| Rémanence : | 23 % |

D'autres bandes élastiques auto adhérentes disponibles dans le commerce ont aussi été utilisées dans le cadre de la présente invention, dont les caractéristiques techniques sont les suivantes :

**Exemple 7**

- Bande élastique auto adhérente commercialisée par la société SMITH et NEPHEW dans le système quatre couches PROFORE® sous la dénomination PROFORE 4®

**Exemple 8**

- Bande élastique auto adhérente commercialisée par la société ACTIVA Healthcare sous la référence ACTICO®

**Exemple 9**

- Bande élastique auto adhérente commercialisée par la société URGO Limited dans le système quatre couches K4 sous la référence KO-FLEX®

Afin d'illustrer l'invention, les différentes bandes décrites dans les exemples 1 à 9 ont été combinées pour réaliser différents systèmes de contention selon l'invention.
Ces combinaisons permettent aussi de mettre en évidence les corrélations qui existent entre le coefficient d'élasticité dynamique appelé en abrégé CED et le différentiel de pression entre pression au repos et pression de travail.
Pour cela, chacune des bandes du système de contention est posée à une extension donnée et on mesure à la pose pour chaque bande et chaque combinaison le CED en dynamométrie et le différentiel de pression appelé ci-après Δ avec l'appareil de tests in vitro.

L'ensemble des résultats obtenus est rassemblé dans le tableau I ci-après dans lequel la première couche du système de contention selon l'invention est référencée sous l'abréviation C1 son CED sous CED1 et son Δ sous Δ1 et la deuxième couche sous l'abréviation C2 son CED sous CED2 et son Δ sous Δ2. De même, l'abréviation CED1 + CED2 représente la somme des CED de chaque bande, l'abréviation Δ1 + Δ2 représente la somme du différentiel de pression de chaque bande, CED (1 + 2) et Δ (1 + 2) représente respectivement les résultats des mesures de CED et de différentiel de pression réalisées pour l'ensemble des deux couches qui forment le système de contention.

Le pourcentage d'élongation à la pose est précisé pour chaque bande.

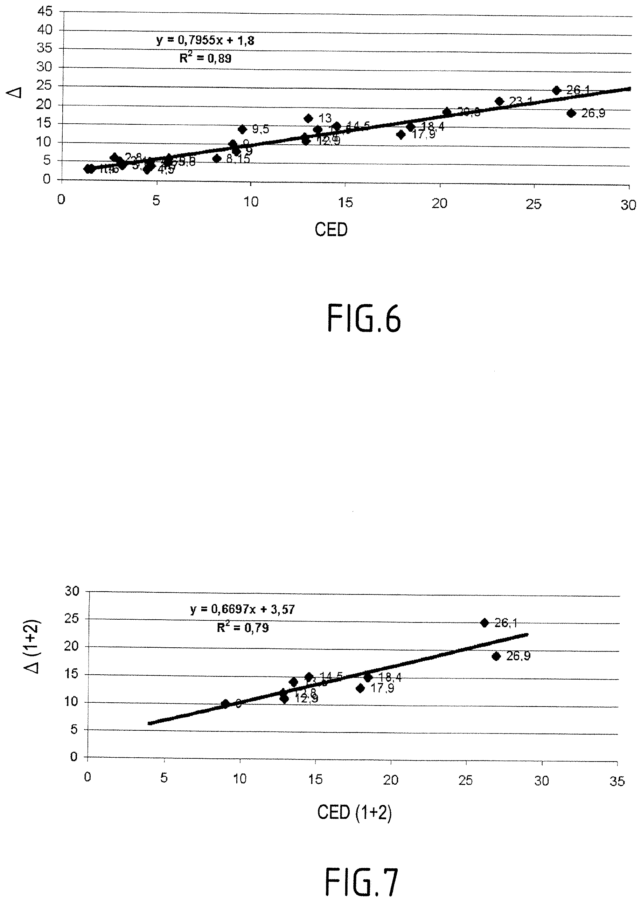

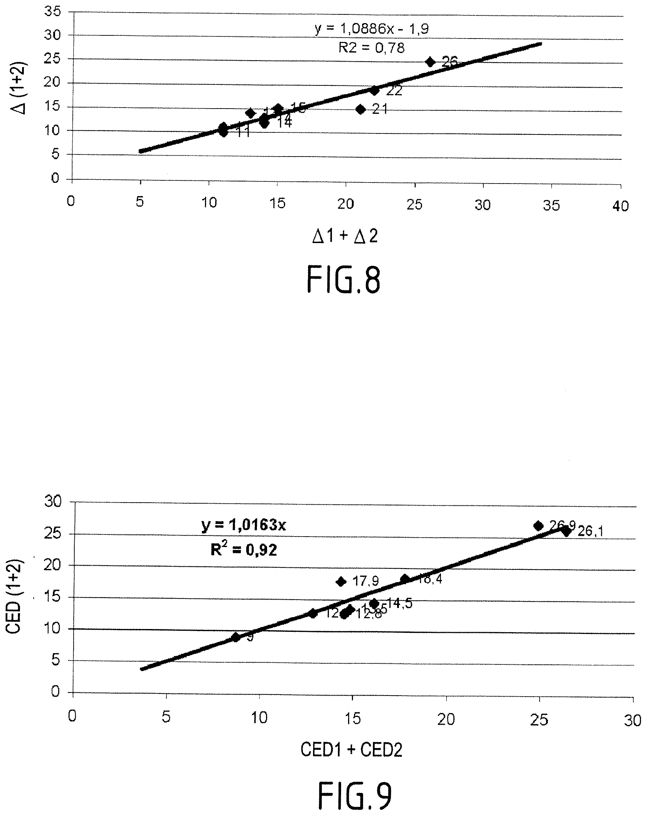

Les figures 6 à 9 illustrent respectivement les courbes de corrélation entre Δ et CED pour l'ensemble des bandes individuelles et leurs associations dans les exemples de système de contention selon l'invention (figure 6), entre Δ et CED uniquement pour les exemples de systèmes de contention selon l'invention (figure 7), entre la somme des Δ de chaque bande individuelle et les Δ mesurés pour les exemples de systèmes de contention selon l'invention (figure 8) et entre la somme des CED de chaque bande et les CED mesurés pour les exemples de systèmes de contention selon l'invention (figure 9).

L'ensemble de ces résultats met en évidence plusieurs caractéristiques importantes.

- Les variations de CED et Δ pour un même produit montre la grande variabilité entre différents lots du même produit textile. Le CED de l'exemple 4 varie ainsi pour un même allongement de 50 % de 3,1 à 5,56.De même, dans l'exemple 2 pour un même allongement de 30 % le CED varie de 4,5 à 8,2. Toutefois, il a été constaté que cette grande variabilité affecte peu les corrélations existantes entre les différents paramètres étudiés.

- A la vue des courbes représentées sur les figures 6 à 9 et des coefficients $R^2$ obtenus respectivement de 0,89 - 0,79 - 0,78 et 0,92 on constate que malgré tous les paramètres (variabilité textile, étalonnage, pose, incertitude sur les mesures), on obtient des corrélations excellentes. Le CED permet donc de s'affranchir de la variabilité des matériaux textiles et constitue donc un paramètre de caractérisation fiable des systèmes de contention selon l'invention.

- Enfin, l'analyse du tableau I montre que les classements des bandes en allongement court, moyen ou long généralement utilisés par les professionnels de santé et les fabricants de bandes de contention ne recouvrent pas ou que très partiellement la réalité et la possibilité d'utilisation de ces produits et en particulier si on considère leur emploi dans un système dynamique. On voit ainsi sur ce tableau que l'on peut utiliser la même bande à des allongements très différents, par exemple 30 à 55 % pour l'exemple 2. De même, on utilise la bande ACTICO dans l'exemple 8 non pas à son allongement maximum tel que recommandé dans son utilisation mais à 60 %. Cette possibilité d'adaptation des bandes pour traiter les pathologies est un élément nouveau et particulièrement avantageux de l'invention.

**Tableau I**

| C1 | C2 | CED1 | CED2 | CED 1 + CED2 | CED (1 +2) | Δ1 | Δ2 | Δ1 + Δ2 | Δ (1 + 2) |
|---|---|---|---|---|---|---|---|---|---|
| EX3 40 % + | EX5 50 % | 4,7 | 13 | 17,7 | 18,4 | 4 | 17 | 21 | 15 |
| EX1 40 % + | EX4 50 % | 5,6 | 3,1 | 8,7 | 9 | 6 | 5 | 11 | 10 |
| EX2 30 % + | EX4 50 % | 8,2 | 4,6 | 12,8 | 12,9 | 6 | 5 | 11 | 11 |
| EX2 40 % + | EX4 50 % | 9,2 | 5,6 | 14,8 | 13,5 | 8 | 5 | 13 | 14 |
| EX2 50 % + | EX4 50 % | 12,9 | 3,2 | 16,1 | 14,5 | 11 | 4 | 15 | 15 |
| EX2 55 % + | EX4 50 % | 23,1 | 3,2 | 26,3 | 26,1 | 22 | 4 | 26 | 25 |
| EX2 50 % + | EX9 50 % | 12,9 | 1,6 | 14,5 | 12,8 | 11 | 3 | 14 | 12 |
| EX2 50 % + | EX7 50 % | 12,9 | 1,4 | 14,3 | 18,9 | 11 | 3 | 14 | 13 |
| EX2 30 % + | EX8 60 % | 4,5 | 20,3 | 24,8 | 26,9 | 3 | 19 | 22 | 19 |

Une bonne connaissance du CED d'une bande permet donc de déterminer de façon précise son action, en particulier en terme de différentiel de pression, et par conséquent de définir le couple de bandes le plus approprié pour réaliser un système de contention apte à traiter efficacement le patient.

Toutefois ceci n'est vrai que dans le domaine de CED défini précédemment. On peut illustrer ceci par le contre exemple suivant.

On a réalisé un système de contention constitué de deux couches correspondant aux exemples 1 et 6, et procédé aux mesures de CED et de Δ, selon les mêmes méthodes que précédemment décrites pour des modalités de pose correspondant à un allongement à 30 % de la première couche (Exemple 1) et à 90 % de la deuxième couche (Exemple 6).

Les valeurs de CED et de Δ de ce système sont les suivantes :

| CED 1 | CED 2 | CED 1 + CED 2 | CED (1 + 2) |
|--------|--------|----------------|--------------|
| 4,7 | 62,8 | 67,5 | 67,6 |
| Δ1 | Δ2 | Δ1 + Δ2 | Δ (1 + 2) |
| 6 | 63 | 69 | 52 |

Au vu de ces résultats, on constate que si la corrélation existe bien entre la somme des CED et les CED de la somme, il n'y a en revanche aucune corrélation entre les Δ de la somme et la somme des Δ (69 contre 52), en dehors du domaine revendiqué.

Ceci confirme que si une des deux bandes possède un CED supérieur à 45 Newtons/cm ou si l'ensemble du système possède un CED supérieur à 55 Newtons/cm, on ne peut prévoir les propriétés du système de contention et en particulier la valeur du différentiel de pression qui est l'élément clé de l'efficacité du système. Bien que les raisons exactes de ce phénomène ne soient pas exactement comprises, il semble que si une des deux bandes est posée à CED trop élevé, ce qui est le cas pour l'exemple 6 à 90 %, elle exerce une force trop importante ce qui entraîne une chute de différentiel de pression importante et immédiate. De plus, si cette chute se poursuit au cours du temps, elle peut conduire à une chute du système de contention. Enfin, les pressions appliquées peuvent être dangereuses car beaucoup trop élevées au départ.

Ceci est mis en évidence par la mesure des pressions de repos, de travail et de différentiel dudit système de contention et de la bande de l'exemple 6 seule à l'aide de l'appareil de tests in vitro.

On obtient ainsi les résultats suivants :

**Exemple 6 à 90 % :**

| | |
|---|---|
| Pression de travail à la pose | : 76 mm de mercure |
| Pression de travail au bout d'une heure | : 44 mm de mercure |
| Δ à la pose | : 63 mm de mercure |
| Δ au bout d'une heure | : 41 mm de mercure |

**Exemple 1 à 30 % + exemple 6 à 90 %**

| | |
|---|---|
| Pression de travail à la pose | : 79 mm de mercure |
| Pression de travail au bout d'une heure | : 55 mm de mercure |
| Δ à la pose | : 52 mm de mercure |
| Δ au bout d'une heure | : 38 mm de mercure |

On voit que l'on a dès la première heure, une baisse très importante des valeurs de pression.

De plus, il y a aussi un risque de garrot avec un tel produit. On constate en effet que les valeurs de pression de travail à la pose de l'ordre de 80 mm de mercure sont aussi très élevées. La mise en place d'une bande élastique auto adhérente telle celle de l'exemple 6 à 90 % pourra donc générer un inconfort durant les premières heures ce qui peut nuire à l'observance du patient qui peut renoncer rapidement à porter un tel système. On peut aussi noter que la pression est appliquée sur le modèle in vitro sur une cheville de 35 cm de diamètre. Or on applique souvent ces systèmes sur des chevilles dont le diamètre moyen est de 23 à 25 cm, de sorte que la pression appliquée sera alors plus importante et les risques et les inconvénients cités précédemment seront augmentés. Enfin, cette bande à une rémanence très élevée de l'ordre de 23 %, elle sera donc très difficile à poser en particulier lors du passage du talon.

On a étudié cette décroissance au-delà d'une heure avec l'appareil de test in vitro décrit précédemment et on a relevé les différentiels de pression de ce système sur plusieurs jours en comparaison avec les produits commerciaux ACTICO® et PROFORE® et avec un système de contention selon l'invention constitué des deux couches suivantes Ex2-40 % + Ex4-50 %.

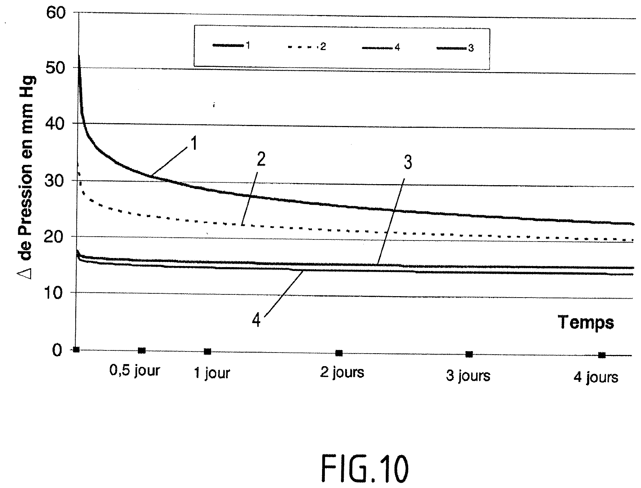

La figure 10 illustre les résultats obtenus. On constate que le système bicouche réalisé avec la bande de l'exemple 6, qui est en dehors des limites fixées selon l'invention en termes de CED, présente une diminution -continue du différentiel de pression (courbe 1). De plus, ce différentiel de pression est au départ très élevé, 52 mm de mercure au lieu d'environ 35 mm de mercure qui est la valeur supérieure recommandée pour l'efficacité.

A l'inverse, le système bicouche selon l'invention (EX2-40 % + EX4-50 %) possède une valeur de différentiel de pression initiale qui ne change quasiment pas et reste constante dans le temps (courbe 3). Elle est comparable au résultat obtenu avec le système quatre couches PROFORE® (courbe 4).

En revanche, on peut noter que le système ACTICO® (courbe 2) présente lui aussi une chute de différentiel même si celle-ci est relativement faible et semble se stabiliser au cours du temps. Ce produit qui est posé à son allongement maximal présente une valeur de différentiel de pression initiale de l'ordre de 29 à 35 mm de mercure qui est acceptable et cette valeur se stabilise rapidement dans le temps. Il présente toutefois une rémanence de l'ordre de 11,5 % à son allongement maximal qui nuit à sa manipulation à la pose. On constate en effet que la pose de cette bande à son allongement maximal est difficile.

Enfin, on peut aussi noter pour ce produit l'importance de l'influence de la ouate qui est posée indépendamment de la bande élastique auto adhérente en comparant les résultats de différentiels de pression entre la bande élastique posée seule et l'ensemble bande + ouate. Lorsque l'on teste ces deux éléments sur l'appareil de tests in vitro, posés à son allongement maximum désigné ci-après 100 %, on obtient les résultats suivants :

|  | ACTICO seul 100 % | ACTICO 100 % + ouate |
|---|---|---|
| Pression de travail à la pose | 74 | 48 |
| Pression au bout d'une heure | 56 | 40 |
| $\Delta$ de pression à la pose | 47 | 29 |
| $\Delta$ au bout d'une heure | 41 | 26 |

On constate à nouveau des variations très importantes qui mettent en avant le rôle potentiel de la ouate.

Tous ces résultats et ces tests démontrent bien l'avantage des systèmes de contention selon l'invention qui permettent d'obtenir un produit efficace avec un différentiel de pression stabilisé rapidement et stable au cours du temps.

Le choix des CED dans les plages de valeurs définies selon l'invention permet aussi d'éviter l'inconfort à la pose et les risques de garrot.

Enfin, même si le système peut présenter une légère diminution du différentiel de pression à la pose dans les valeurs de CED les plus élevées, celle-ci est faible et reste dans les limites qui permettent une utilisation en toute sécurité du produit.

De plus, en choisissant des bandes qui présentent une rémanence inférieure à 10 % et de préférence entre 4 et 8 %, on obtient toujours un produit facile à poser ce qui n'est pas évident dans le cas des produits de l'état de la technique du type ACTICO.

**Revendications**

**1.** Système de contention bicouche exerçant, entre une position de repos dans laquelle le muscle est relâché et une position de travail dans laquelle le muscle est tendu, un différentiel de pression compris entre 10 et 40 mm de mercure, **caractérisé en ce qu'**il comprend :

- une première couche de contention constituée d'une bande élastique liée sur l'ensemble de sa surface à une couche absorbante , ladite couche absorbante étant constituée d'un matériau textile tissé ou non tissé à base de fibres, et ladite bande élastique étant constituée d'un matériau textile tissé, non tissé ou d'un tricot;
- une deuxième couche de contention constituée d'une bande élastique auto adhérente;
- lesdites première et seconde couches étant en outre pourvues d'un système d'étalonnage indiquant l'allongement à la pose respectif de chacune de ces deux couches et choisi de telle sorte que :

le coefficient d'élasticité dynamique desdites première et deuxième couche soit compris entre 1 et 45 Newtons/cm et que
le coefficient d'élasticité dynamique du système de contention soit compris entre 5 et 55 Newtons/cm;
le coefficient d'élasticité dynamique étant mesuré selon la description;

- lesdites première et deuxième couche présentant en outre une rémanence à leur allongement à la pose inférieure à 10 %.

2. Système de contention bicouche selon la revendication 1 **caractérisé en ce que** le coefficient d'élasticité dynamique du système est compris entre 10 et 35 et de préférence 15 et 30 Newtons/cm.

3. Système de contention bicouche selon les revendications 1 ou 2 **caractérisé en ce que** la première ou la deuxième couche possède un coefficient d'élasticité dynamique compris entre 3 et 30 et de préférence entre 5 et 25 Newtons/cm.

4. Système de contention bicouche selon l'une des revendications précédentes **caractérisé en ce que** la première couche est constituée d'une ouate liée par aiguilletage à un tricot.

5. Système de contention bicouche selon l'une des revendications précédentes **caractérisé en ce que** la première et /ou la deuxième couche présente une rémanence à son allongement à la pose comprise entre 4 et 8%.

6. Système de contention bicouche selon l'une des revendications précédentes **caractérisé en ce que** le moyen d'étalonnage précité est constitué de pictogrammes.

**Patentansprüche**

1. Zweischicht-Stützsystem, das zwischen einer Ruhestellung, in der der Muskel entspannt ist, und einer Arbeitsstellung, in der der Muskel angespannt ist, eine Druckdifferenz ausübt, die zwischen 10 und 40 mm Quecksilber liegt, **dadurch gekennzeichnet, dass** es umfasst:

   - eine erste Stützschicht, die aus einem elastischen Band besteht, das über seine gesamte Fläche mit einer absorbierenden Schicht verbunden ist, wobei die absorbierende Schicht aus einem gewebten oder nicht gewebten Textilmaterial auf Faserbasis besteht und das elastische Band aus einem gewebten oder nicht gewebten Textilmaterial oder einem Strickgewebe besteht,
   - eine zweite Stützschicht, die aus einem selbsthaftenden elastischen Band besteht,
   - wobei die erste und die zweite Schicht ferner mit einem Kalibrierungssystem versehen sind, das die Dehnung von jeder dieser zwei Schichten bei ihrem jeweiligen Anlegen angibt und derart gewählt ist, dass:

      der dynamische Elastizitätskoeffizient der ersten und der zweiten Schicht zwischen 1 und 45 Newton/cm liegt und dass
      der dynamische Elastizitätskoeffizient des Stützsystems zwischen 5 und 55 Newton/cm liegt,
      der dynamische Elastizitätskoeffizient gemäß der Beschreibung gemessen wird,

   - die erste und die zweite Schicht ferner bei ihrer Dehnung beim Anlegen eine bleibende Verformung von weniger als 10% aufweisen.

2. Zweischicht-Stützsystem nach Anspruch 1, **dadurch gekennzeichnet, dass** der dynamische Elastizitätskoeffizient des Systems zwischen 10 und 35 und vorzugsweise 15 und 30 Newton/cm liegt.

3. Zweischicht-Stützsystem nach Anspruch 1 oder 2, **dadurch gekennzeichnet, dass** die erste oder die zweite Schicht einen dynamischen Elastizitätskoeffizienten aufweist, der zwischen 3 und 30 und vorzugsweise zwischen 5 und 25 Newton/cm liegt.

4. Zweischicht-Stützsystem nach einem der vorhergehenden Ansprüche, **dadurch gekennzeichnet, dass** die erste Schicht aus einer Watte besteht, die durch Nadeln mit einem Strickgewebe verbunden ist.

5. Zweischicht-Stützsystem nach einem der vorhergehenden Ansprüche, **dadurch gekennzeichnet, dass** die erste und/oder die zweite Schicht bei ihrer Dehnung beim Anlegen eine bleibende Verformung aufweist/en, die zwischen 4 und 8 % liegt.

6. Zweischicht-Stützsystem nach einem der vorhergehenden Ansprüche, **dadurch gekennzeichnet, dass** das vorhergehend genannte Kalibrierungsmittel aus Piktogrammen besteht.

**Claims**

1. Two-layer compression system exerting, between a resting position where the muscle is relaxed and a working position where the muscle is tensed, a pressure difference between 10 and 40 mm of mercury, **characterized in that** it comprises:

   - a first compression layer consisting of an elastic bandage linked over the whole of its surface with an absorbent layer, said absorbant layer consisting of a woven or non-woven fibre-based textile material and said elastic bandage consisting of a woven or a non-woven textile material or a knit;
   - a second compression layer consisting of a self-adhesive elastic bandage;
   said first and second layers being further provided with a calibration system indicating the stretch at application respective to each of these two layers and chosen so that:

   the dynamic elasticity coefficient of said first and second layers is between 1 and 45 newtons/cm; and
   the dynamic elasticity coefficient of the compression system is between 5 and 55 newtons/cm
   the dynamic elasticity coefficient being measured according to the description;

   - said first and second layers further having a tension set measured for their stretch on application of less than 10%.

2. Two-layer compression system according to Claim 1, **characterized in that** the dynamic elasticity coefficient of the system is between 10 and 35 and preferably between 15 and 30 newtons/cm.

3. Two-layer compression system according to Claims 1 and 2, **characterized in that** the first or the second layer has a dynamic elasticity coefficient of between 3 and 30 and preferably between 5 and 25 newtons/cm.

4. Two-layer compression system according to one of the preceding claims, **characterized in that** the first layer consists of a padding linked by needling to a knit.

5. Two-layer compression system according to one of the preceding claims, **characterized in that** the first and/or the second layer has a tension set, measured for their stretch on application, of between 4 and 8%.

6. Two-layer compression system according to one of the preceding claims, **characterized in that** said calibration means is in the form of pictograms.

F en Newtons/cm

FIG.1

Déformation (%)

F en Newtons/cm

FIG.3

$F_{Max}$
$F_{Min}$

Allongement en %

$\varepsilon_{Min}$    $\varepsilon_{Pose}$    $\varepsilon_{Max}$

FIG.2

EP 1 998 730 B1

1    2

$L_0(1+E_1)$

$L_0$    $L_0(1+\alpha)$

1    2

$L'_0(1+E_2) \Rightarrow L'_0 = L_0 \times \left( \dfrac{1+E_1}{1+E_2} \right)$

## FIG.4

F en Newtons/cm

Energie
cycle

$\varepsilon_r$ :  Déformation
Rémanente

$\varepsilon_{Pose}$    Allongement en %

## FIG.5

FIG.6

FIG.7

FIG.8

FIG.9

FIG.10

**EP 1 998 730 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0878179 A
- US 20050192524 A
- EP 490793 A
- EP 671898 A
- EP 820261 A
- EP 876809 A
- US 3613679 A
- EP 460040 A
- EP 651628 A
- EP 0873097 A
- US 4699133 A
- FR 2611756